# EUROPEAN PATENT SPECIFICATION

(11) **EP 2 059 292 B1**
(45) Date of publication and mention of the grant of the patent: **11.11.2015**
(21) Application number: 07842253.2
(22) Date of filing: 11.09.2007
(51) Int. Cl.: A61F 2/01

(54) **EMBOLIC PROTECTION DEVICE**
EMBOLIESCHUTZVORRICHTUNG
DISPOSITIF DE PROTECTION CONTRE LES EMBOLES

(30) Priority: 11.09.2006 US 518865
(43) Date of publication of application: 20.05.2009
(73) Proprietor: Embrella Cardiovascular, Inc., Wayne, PA 19087 (US)
(72) Inventor: CARPENTER, Judith T., Radnor, PA 19087 (US)
(74) Representative: Gill, David Alan
(86) International application number: PCT/US2007/078170
(87) International publication number: WO 2008/033845

(56) References cited:
- WO-A2-2006/076505
- US-A- 5 695 519
- US-A- 5 769 816
- US-A- 6 027 520
- US-A1- 2002 128 679
- US-A1- 2003 120 304
- US-B1- 6 258 120
- US-B1- 6 395 014
- US-B1- 6 673 089

## Description

### Background of the Invention

Endovascular procedures are being used more and more frequently to treat various cardiac and vascular surgical problems. Blocked arteries can be treated with angioplasty, endarterectomy, and/or stenting, using minimally invasive endovascular approaches. Aneurysms can be repaired by endovascular techniques. Another use for endovascular surgery is the treatment of cardiac valvular disease. Valvuloplasties are already being done endovascularly and percutaneous valve replacement will surely follow, as it has already been tested in Europe. A major problem which is common to all these endovascular manipulations is that plaque found in the diseased vessels and valves can be dislodged and result in embolization. A major drawback to endovascular treatment of cardiac valves and arteries in the heart or thoracic aorta is that the dislodged debris can embolize into the carotid vessels resulting in catastrophic consequences such as stroke or even death. Attempts have been made to protect the cerebral vasculature with filters and other devices, but the inadequacy of the present art is obvious in the fact that these devices are rarely used. The plethora of pending patent applications for such protection devices suggests both the inadequacy of the present art and the need for improved devices.

The majority of devices described are filters. The problems with filters include difficulty in placement and retrieval as well as the possibility that a filter will fill abruptly causing blockage of the artery prior to removal of the filter. Cerebral protection requires placement of filters in the carotid arteries, which has the additional drawback of manipulation of the carotid vessels during filter placement while the cerebral vasculature is still unprotected. The risk of stroke for a carotid arteriogram done by cannulation of the carotid artery is 1% compared to an arteriogram done from injection into the aorta without selective cannulation which carries minimal risk. The risk of cannulating a carotid artery, navigating a catheter containing a filter into position, and deploying the filter would likely carry an even higher stroke risk. Patients requiring cardiac or aortic arch procedures are high risk candidates for having carotid disease. The chance of causing a stroke by the placement of a protective device into both carotid arteries makes the risk of using these devices prohibitive. The time and skill necessary to selectively cannulate both carotid arteries for filter placement has also contributed to the decision not to use them despite the stroke risk of unprotected cardiac and aortic arch procedures.

US6027520 discloses a filter configured to be percutaneously advanced into the placed into blood vessels such as the aorta, with the filter positioned entirely across the blood vessel so that all blood flowing through the blood vessel must pass through the filter.

US2002128679 discloses a shunt and method of use for maintaining distal blood flow during an arteriotomy procedure. The shunt includes first and second tubular members having proximal ports, distal ports, and lumens therebetween. The distal port of the second tubular member is adapted for releasable attachment to the proximal port of the first tubular member. A second lumen merges and communicates at its distal end with the lumen of the first tubular member and includes a hemostatic valve attached to its proximal end. In using the apparatus for performing open endarterectomy, a filter device is inserted into the vessel and deployed downstream the region of interest in the internal carotid artery. The distal end of the shunt is advanced over the filter device and secured onto the artery. The proximal end of the shunt is inserted upstream the region of interest, typically in the common carotid artery.

US2003120304 discloses a method for carotid endarterectomy. A blood filter is positioned within a carotid artery downstream of an atheromatous plaque. The filter is expanded. The carotid artery is clamped upstream and downstream of the atheromatous plaque while the filter is expanded. An endarterectomy procedure is performed on the region of the carotid artery having the atheromatous plaque. The carotid artery is undamped while the filter is expanded. In certain cases a shunt is positioned in a region of the carotid artery having the atheromatous plaque and secured to the shunt by clamping, the shunt comprising a tubular member having a proximal opening, a distal opening, and a lumen therebetween.

WO2006076505 discloses a method for treating both sessile and mobile aortic atheroma. A radially expanding device, such as a stent or compliant cast, comprising a generally cylindrical member expandable between a compressed state and an enlarged state is provided.

US5695519 discloses a distal intravascular filter for filtering blood flow therethrough and entrapping and retaining embolic debris. The intravascular filter including a small diameter hollow guide wire or tube capable of percutaneous placement of the distal end thereof beyond a carotid stenosis. The distal portion of the tube includes a filter mounted thereon. The filter is deployable from a tightly closed configuration to an open circumference for blocking the unfiltered flow of blood beyond the carotid stenosis. The filter is deployable between open and closed positions by manipulation of an actuating wire extending from the filter and out the proximal end of the tube.

### Brief Description of the Invention

The present invention comprises a deflector umbrella. In use, the invention is placed into the aortic arch by the Seldinger technique, preferably through the right arm but alternatively via the femoral artery. Alternatively, it could be placed directly into the aorta in the case of an open cardiac or aortic procedure. It is deployed in the aortic arch, where the device is opened and preferably placed into position to cover the ostia of both the brachiocephalic and left common carotid arteries.

According to an aspect of the present invention, there is provided an embolus deflector for protecting against cerebral embolization comprising a blood flow permeable covering configured to extend over either (a) the ostia of the right common carotid and left common carotid arteries, or (b) the ostia of the brachiocephalic and the left common carotid arteries; characterized in that the deflector comprises an expandable support frame, wherein the support frame comprises multiple petals, wherein each petal is formed from wire, wherein porous material extends between the wire of the petals to form the blood flow permeable covering; wherein the support frame has a closed configuration wherein the petals are restrained and an open configuration wherein the petals are opened, wherein in the closed configuration the support frame is sized and configured to be introduced into the aorta, and the support frame is sized and configured to be converted from the closed configuration to the open configuration within the aorta, wherein in the open configuration the support frame is sized and configured to support the blood flow permeable covering within the aorta in a position within the aortic arch and against the aortic wall such that the blood flow permeable covering extends over and covers the left common carotid artery ostium while simultaneously extending over and covering at least one of the ostia of the brachiocephalic artery or of the right common carotid artery, wherein the blood flow permeable covering simultaneously presents a low profile against the aortic wall and within the lumen of the aortic arch to thereby cause minimal resistance to blood flow in the aortic arch by permitting blood to flow through the aortic arch past the blood flow permeable covering without passing therethrough and also to allow sheaths, catheters, or wires to pass through the aortic arch and past the blood flow permeable covering without passing therethrough..

### Brief Description of the Figures

**Fig. 1** depicts brachial artery insertion of the deflector of the present invention.
**Fig. 2** depicts femoral artery insertion of the deflector of the present invention.
**Figs.3A-E** depict the preferred method of deployment of the deflector of the present invention through the patient's right arm, thus allowing the deflector to be pulled back against the aortic wall to place it.
**Figs.4A-F** depict an alternative method of deployment of the deflector of the present invention through the femoral artery wherein the deflector is pushed against the aortic wall over the brachiocephalic and left common carotid openings.
**Figs. 5A-L** **and** **5AA-KK** depict various conformations of the deflector of the present invention in plan view (5A-H), phantom plan view (5I-L) and side view (5 AA- KK).
**Figs. 6A-D** depict various conformations of the locking mechanism of the present invention.
**Figs. 7A-C** depict another embodiment of the deflector of the present invention comprising a coil support which expands and flattens upon emergence from the lumen of a tubular containing structure.
**Figs. 8A-C** depict yet another embodiment of the deflector of the present invention comprising a helical, spherical, or onion-shaped mesh that flattens into a disc shape upon emergence from the lumen of a tubular containing structure.

### Detailed Description of the Invention

The deflector ("umbrella" or other conformation) of the present invention is positioned prior to any manipulation of the heart or thoracic aorta. It is simple to place and carries only the minimal risk of catheterizing the aorta through the arm or leg. The device is opened in the thoracic aorta and positioned to cover the ostia of both the brachiocephalic and left common carotid arteries. This position prevents emboli from entering the cerebral circulation through either the right or left carotid arteries with one simple device. Any emboli from the cardiac or aortic procedure are deflected downstream. The device, due to its low profile in the aortic lumen, allows passage of catheters, sheaths, or wires used in the index procedure. After the procedure is complete, the device is inverted or closed by means of a sheath extended over the catheter wire, which then wholly or partially covers the device prior to withdrawal. Should any clot or debris be attached to the outer side of the device, it will be captured in the inverted or closed device and withdrawn. One advantage of the device is that one size fits all, so it can be kept available in stock.

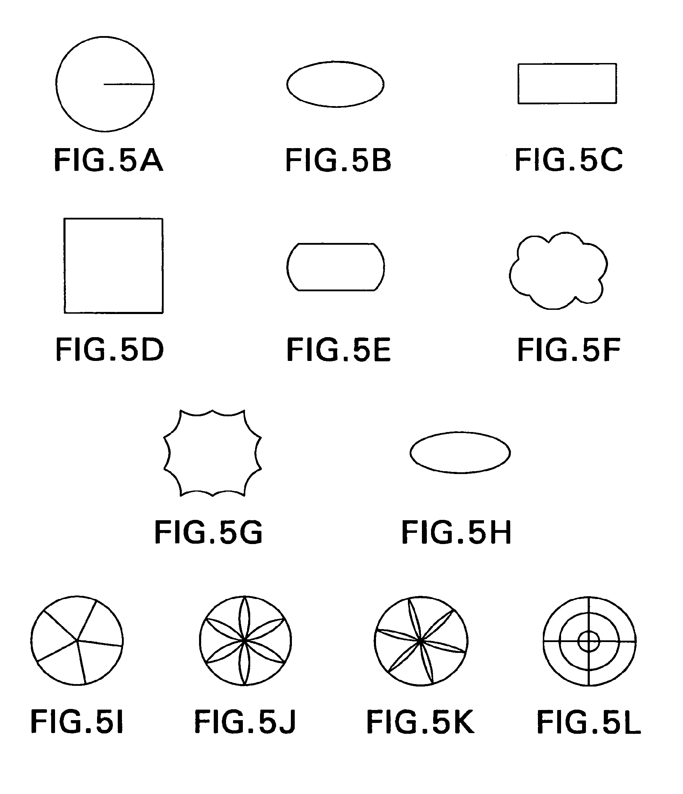

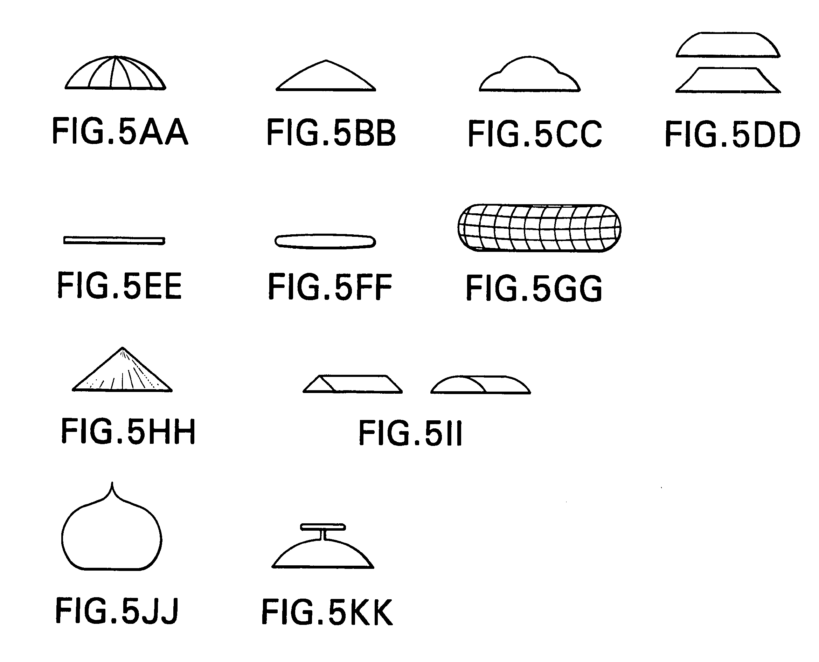

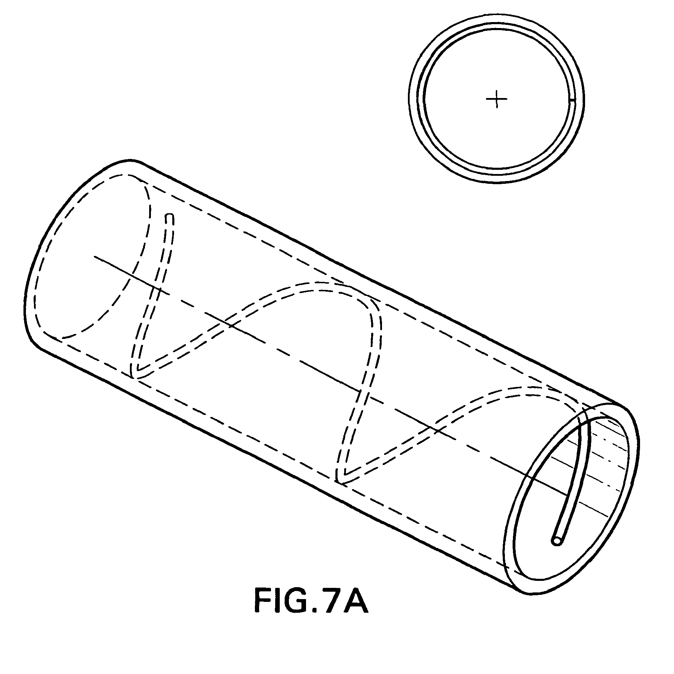

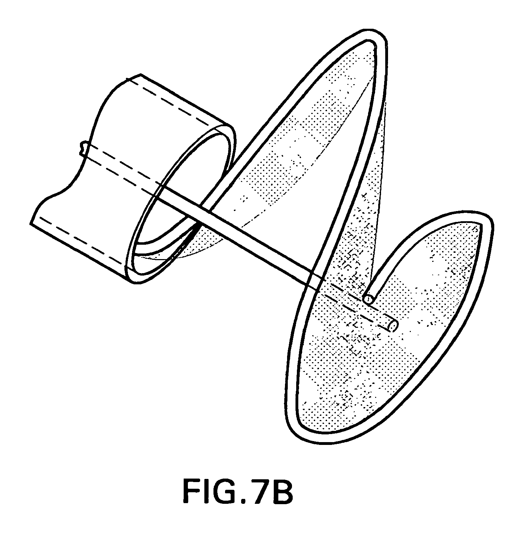

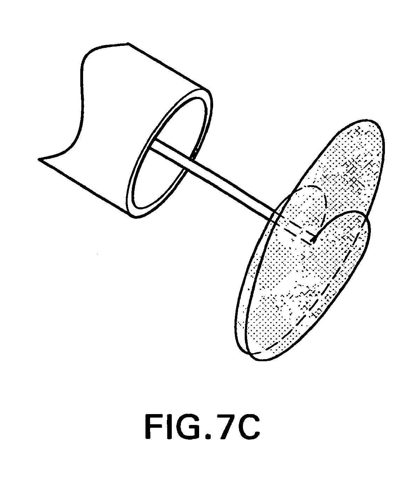

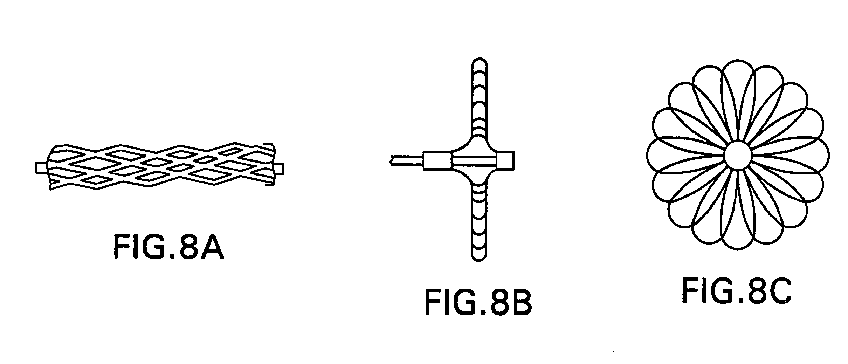

A preferred embodiment of the device is an umbrella-type structure which is preferably dome-shaped with an adequate diameter of from about 20mm to about 40mm, and preferably about 30mm, to cover the ostia of both the brachiocephalic and left common carotid arteries, made of a material with pores or similar openings or permeability to allow the flow of blood into the cerebral circulation, but able to deflect and/or trap emboli of a size which could cause a stroke (as depicted in Figs. 5A-X, 7A-C, 8A-C.) The edge of the umbrella is preferably a flexible, porous toroidal shape, similar to the edge of a common vaginal diaphragm, allowing a good seal with the curved aortic wall. The edge will preferably contain a nitinol or other suitable wire ring. The dome part of the umbrella preferably has struts or ribs to assist in the opening and closing of the umbrella and to help maintain its position. The center of the umbrella preferably has a knob or similar projection on the surface, to which the struts are attached. This area may also contain a swivel mechanism or similar angularly flexible link to allow good positioning and seal of the device with the aortic wall. The deflector is pushed out of the delivery catheter, having an internal diameter of preferably about 1.5mm to about 3mm, with a tube that engages this knob. This knob helps with the opening of the umbrella. The knob remains attached to the umbrella "handle", and a guide wire is used to pull the umbrella into position. The device may also be made to open as a result of its construction material, for example, nitinol or polymer, elastically resuming its shape after being released from its sheath. The device may also open as the result of release from the delivery catheter of a number of wires that represent the struts of the umbrella with porous material bridging the struts. Alternatively, the end of the catheter itself may separate into supporting struts when the device is deployed.

When the umbrella is to be closed, a tube or sheath of larger diameter than the knob is extended over the guide wire until it engages the knob. The umbrella is pulled back so that it inverts and is enclosed in the tube for removal. Inverting the device assures that no trapped or adhered emboli escape into the bloodstream. The device is preferably constructed of polymer, fabric, metal, or a combination of these materials.

The device may also optionally be equipped with radio-opaque markers or other structural parts which are radio-opaque for aid in placement guidance.

Another embodiment of the device has a rolled edge.

The device may also have a flat porous edge.

Another embodiment of the device has no struts, but instead has a nitinol skeleton.

Another embodiment has multiple wires to position and anchor the device.

Another embodiment of the device has anchors at the edges which help to maintain its position during the procedure.

Another embodiment of the device is parachute-like, with a ring gasket at its edge. The gasket is held firmly in position over the ostia of the brachiocephalic and left common carotid arteries. The billowy porous middle section would deflect or trap clot and debris on its exterior surface while causing minimal resistance in the aorta. The middle portion is inverted as it is removed by pulling on wires attached to its center, capturing any clot stuck to it.

Alternatively, the center of the device may comprise a screen, which fits more snugly against the aortic wall, with a very small profile, further preventing resistance to downstream aortic blood flow. Again the device would be removed by inversion, capturing any debris stuck to it prior to removal.

Another embodiment of the device is barbell shaped, with either a porous balloon or porous filter on opposite ends, or a porous sausage shape that inflates along its long axis. Debris is thus deflected downstream in the blood flow by the mid-section of the device which protrudes into the aortic lumen when the device is properly placed. The porous balloon of the distal end may also be substituted with another shape such as a disc or dome such that the balloon protects the brachiocephalic artery, and the distal portion protects the ostium of the left common carotid artery.

As depicted in Figs. 5A-X, and 7A-C, 8A-C the shape of the device may be oval or rectangular or of another shape to assist in sealing of the edge against the wall of the aorta, covering the ostia of both the brachiocephalic and left common carotid arteries and maintaining a low profile within the lumen of the aorta. The device may have petals or other parts which come together to make the deflector shape.

In profile, the device may be flat, concave, or convex with a shape which may be a rounded dome, a tent, a pyramid, a cone, a plateau, or a disc with more than one layer.

This device could be modified in size in another embodiment in order to be used to cover the ostia of different vessels.

The device may be coated with something which prevents clots (e.g., heparin).

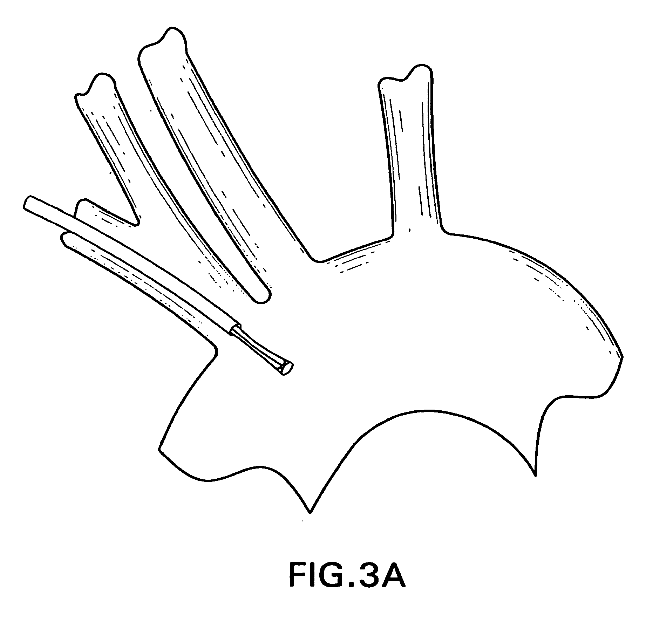

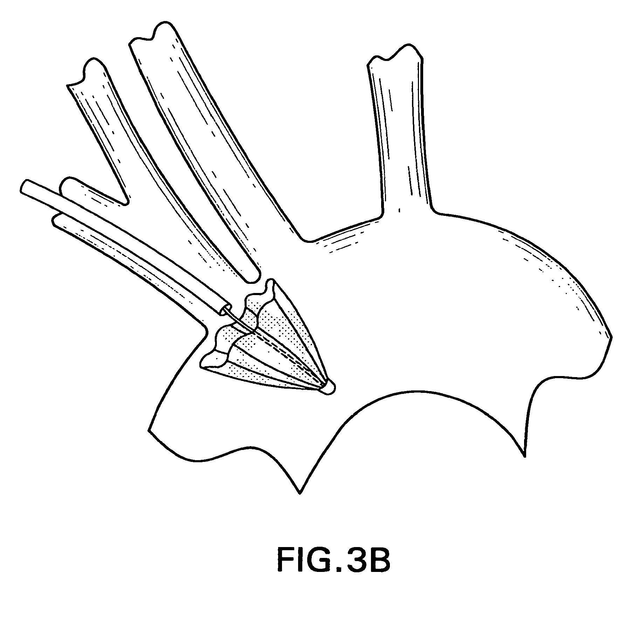

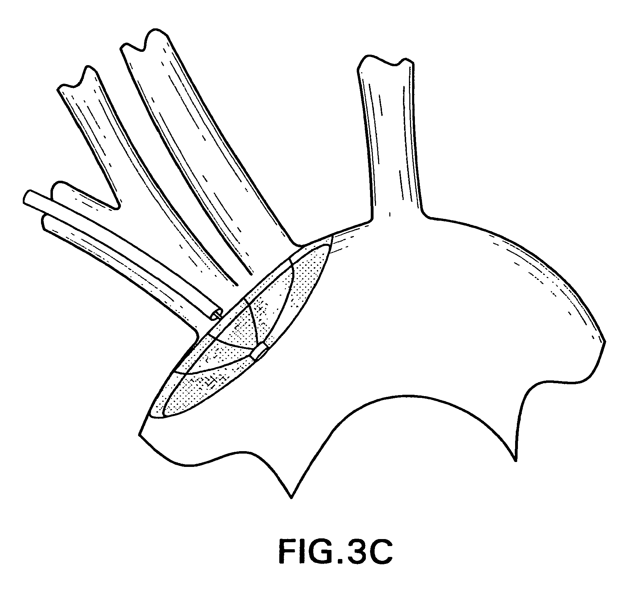

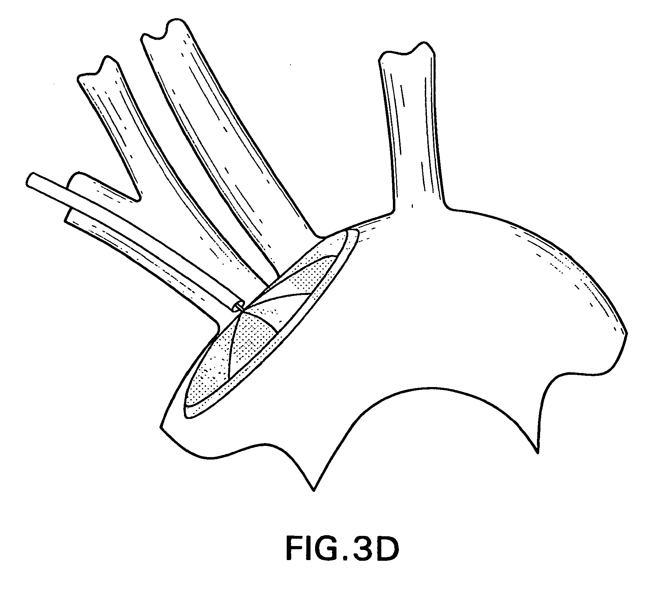

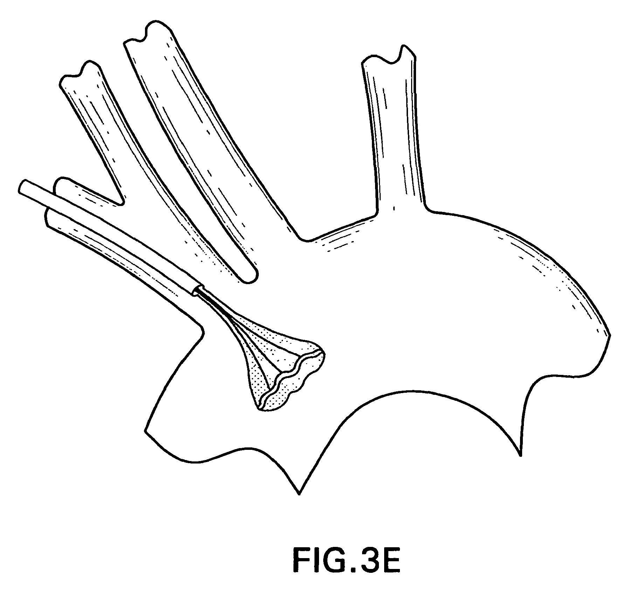

The device may be deployed through an artery of the arm, or through the femoral artery. The preferred method would be through the right arm, if possible, as this would allow the device to be pulled back against the aortic wall to place it (Fig. 3).

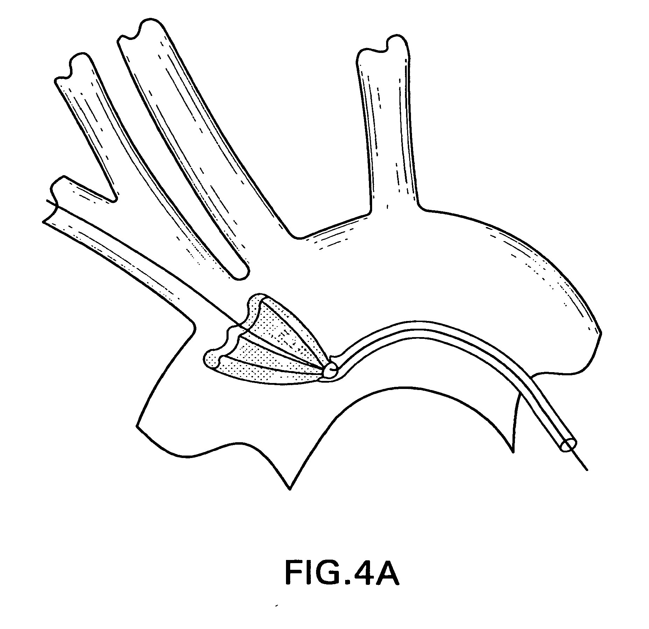

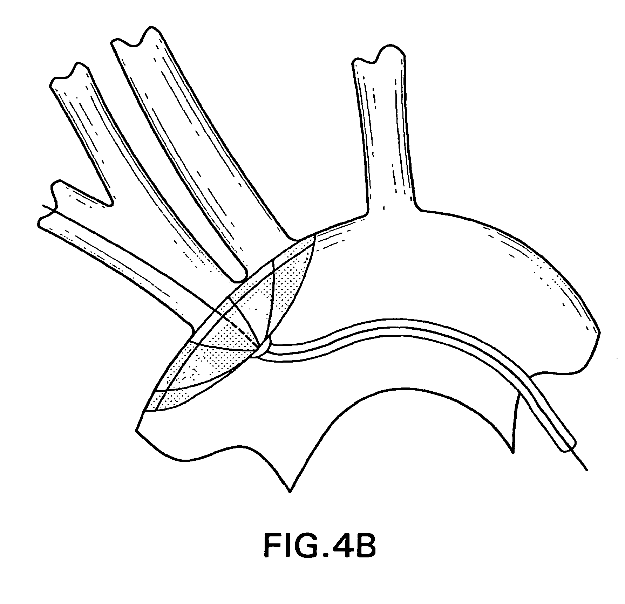

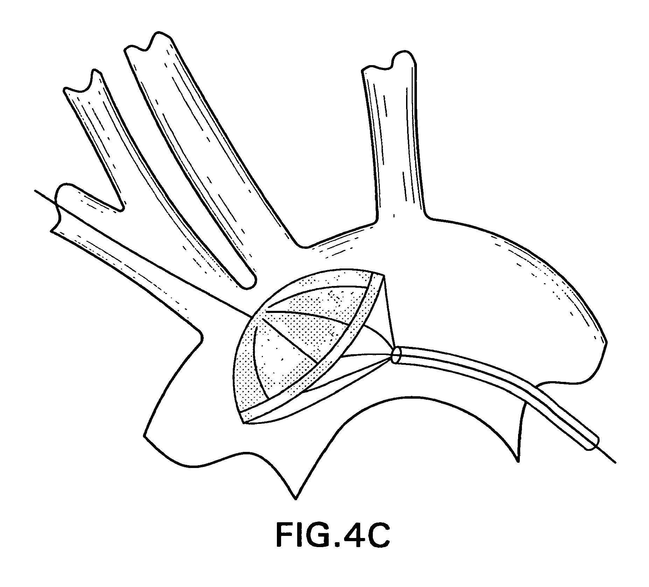

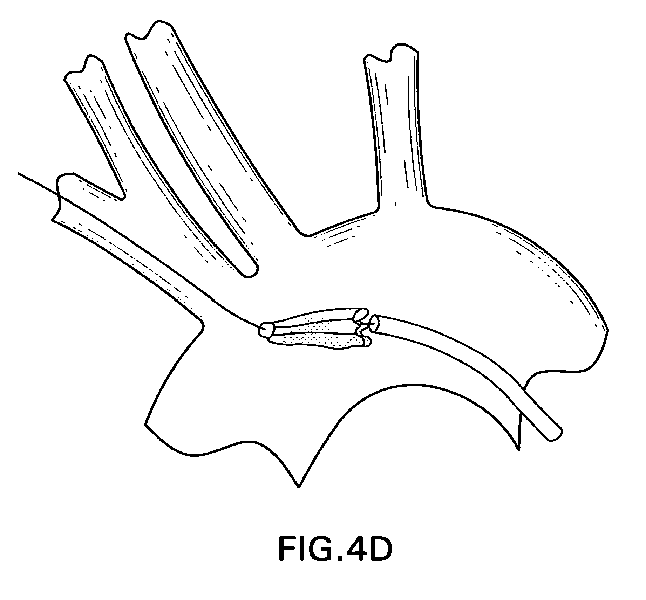

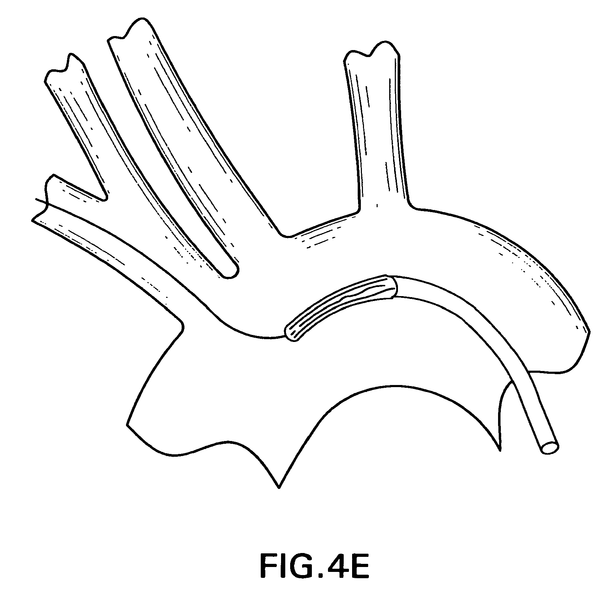

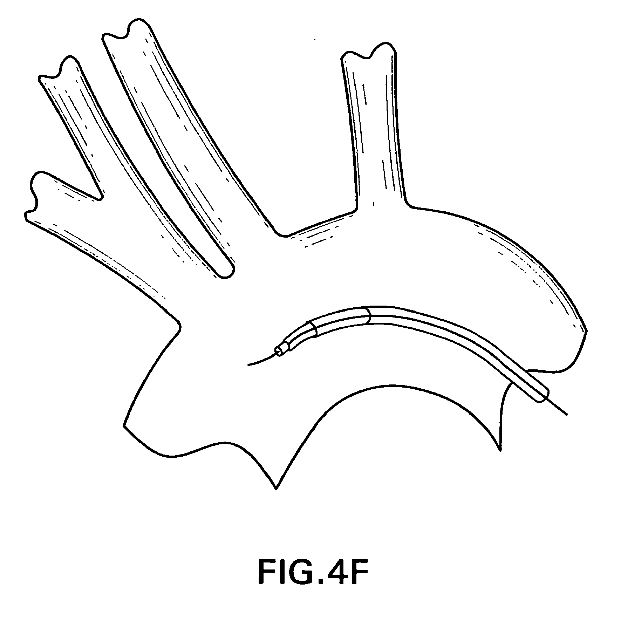

When deployed through the femoral artery (Fig. 4), the opening of the umbrella would be different, and the umbrella would be pushed against the aortic wall over the brachiocephalic and left common carotid openings rather than being pulled back. A wire would be cannulated into the brachiocephalic artery in this case to ensure correct positioning of the device. The device would be modified to allow this method of delivery and positioning. In this embodiment, the knob would be on the outside of the umbrella and the handle would be a firm catheter to allow pushing. In this case, retrieval of the device would involve inversion and closing of the "umbrella" by drawstring or another method.

The device may also be deployed into a position in which one edge is inside the brachiocephalic artery, covering the ostium of right common carotid, and in which the opposite edge extends into the aortic lumen and covers the ostium of the left common carotid artery.

### Brachial artery insertion of the device:

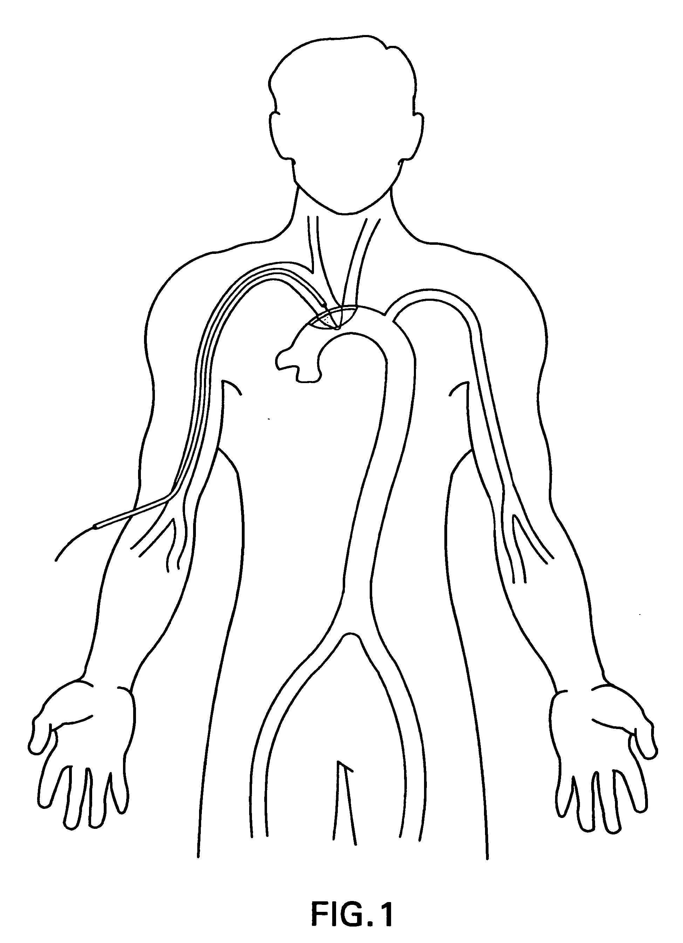

Referring now to Fig. 1, the deflector is delivered via percutaneous insertion into the right brachial artery and is guided into the aortic arch. There it is deployed and then pulled back into position to cover the ostia of the innominate and left common carotid arteries. The device deflects emboli during aortic and cardiac procedures, allowing the flow of blood through into the cerebral circulation (carotid arteries) but not permitting the passage of emboli of a size which could cause stroke.

### Femoral artery insertion of the device:

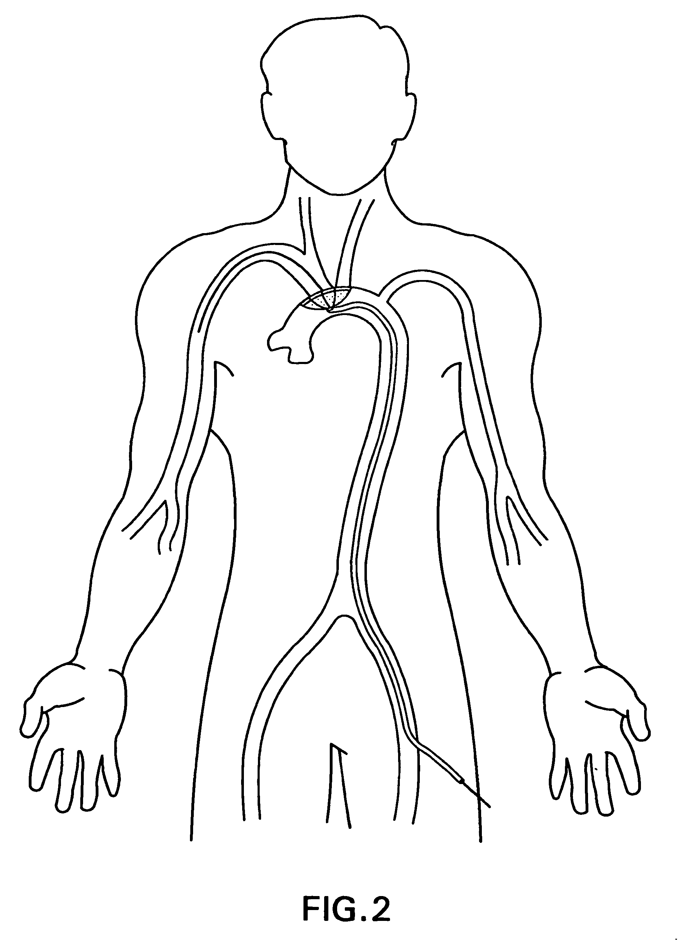

Referring now to Fig. 2, the deflector is delivered via percutaneous insertion into the femoral artery and is guided into the aortic arch. After catheterization of the innominate artery, the device is passed over the wire and brought into position covering the ostia of the innominate and left common carotid arteries.

### Deployment of the device via arm approach:

Referring now to Figs. 3A-E, percutaneous access to the circulation via the right arm (through any appropriate artery) is performed and a wire guided into the aortic arch after exiting the innominate artery. The device is placed over the wire and guided into the aortic arch. The covering outer sheath which encapsulates the device is retracted (Fig. 3A), exposing the device to the aortic bloodstream. The device is then opened in the aortic arch (Fig. 3B). The device is pulled back into position, covering the ostia of both the innominate and left common carotid arteries. The device allows the passage of blood through to the carotid arteries, but deflects emboli generated by aortic or cardiac surgery away from these arteries. At the completion of the debris producing concomitant procedure, the device is closed by inverting the covering cap (Fig. 3D). The device is then withdrawn into a covering sheath (Fig. 3E) to completely encapsulate it prior to removal from the arm access artery. Any trapped debris is enfolded within the closed cap, safely and securely within the covering sheath.

### Deployment of the device via femoral approach.

Referring now to Figs. 4A-F, the innominate artery is catheterized with a wire placed via femoral access. Over the wire, the device is guided into position in the aortic arch, where it is deployed by unsheathing (Fig. 4A). The device is then pushed, over the wire in the innominate artery, into position securely covering the ostia of the innominate and left common carotid arteries (Fig. 4B). The device allows the passage of blood through to the carotid arteries, but deflects emboli generated by aortic or cardiac surgery away from these arteries. At the completion of the debris producing concomitant procedure, the device is closed by inverting the covering cap (Fig. 4C), shown here by means of drawstrings. The device is then collapsed (Fig. 4D) and withdrawn into a covering sheath (Fig. 4E) to completely encapsulate it prior to removal from the leg access artery. Any trapped debris is enfolded within the closed cap, safely and securely within the covering sheath. The wire and device are then withdrawn from the femoral access.

### Direct Insertion of the device:

The device could also be used with open cardiac or aortic procedures. In these cases, the device could be placed as above or directly into the aorta if the arch were exposed. If it were placed directly, it would be pushed into place as with the femoral approach. Alternatively, any appropriate surgical, percutaneous, or endoscopic procedure may be employed to place the device.

### Embolic deflecting device:

Referring now to Fig. 5A, the deflector of the present invention, viewed from above, is dome-shaped with an adequate diameter to cover the ostia of both the brachiocephalic and left common carotid arteries, of about 20mm to about 40mm, and preferably about 30mm, made of a material with pores to allow the flow of blood, but deflect or trap emboli of a size which could cause a stroke. The edge of the umbrella is a flexible, porous donut, similar to the edge of a diaphragm, allowing a good seal with the curved aortic wall. The edge will preferably contain a nitinol wire ring. The dome part of the umbrella has struts to assist in the opening and closing of the umbrella and to help maintain its position.

The center of the umbrella has a knob on the inside surface to which the struts are attached. The device is pushed out of the delivery catheter with a tube, wire or other member that engages this knob. This knob helps with the opening of the umbrella. The knob remains attached to the umbrella "handle", the guide wire is used to pull the umbrella into position. The device may also open as a result of the material it is made of, e.g., nitinol or a suitable polymer, resuming its shape after being released from its sheath. The device may also consist of wires which assume their curved dome shape as they are released from the catheter. The porous fabric between the wires is attached at the highest point of the profile to assist with an umbrella-like deflection of clot or debris. The catheter itself may divide at its distal end to comprise the struts of the umbrella deflector. A single wire may be shaped into petal-like struts for the deflector which assume their umbrella shape upon exit from the delivery catheter.

The device is constructed of polymer, fabric, metal, or a combination of these materials. The device may be provided with radioopaque markers or metal parts which are radioopaque.

Another embodiment of the device has a rolled edge. The device could also have a flat porous edge. Another embodiment of the device has no struts, but a nitinol skeleton. Another embodiment has multiple wires to position and anchor the device. Another embodiment of the device has anchors at the edges which help to maintain its position during the procedure.

Another embodiment of the device is parachute-like, with a ring gasket at its edge. The gasket would be held firmly in position over the ostia of the brachiocephalic and left common carotid arteries. The billowy porous middle section would deflect or trap clot and debris on its exterior surface while causing minimal resistance in the aorta. The middle portion would be inverted as it is removed by pulling on wires attached to its center, capturing any clot stuck to it. Alternatively, the center of the device could be a screen, which fits more snugly against the aortic wall, with a very small profile, further preventing resistance. Again the device would be removed by inversion, capturing any clot stuck to it prior to removal.

Another embodiment of the device comprises a rib-supported or self-supporting spherical shape covered by porous material, which may be distorted into a flat or semi-flat shape for covering the arterial ostia by withdrawing a wire attached to one side of the sphere.

The device may be oval or rectangular or of another shape to assist in sealing of the edge against the wall of the aorta, covering the ostia of both the brachiocephalic and left common carotid arteries and maintaining a low profile within the lumen of the aorta. This device could be modified in size in another embodiment in order to be used to cover the ostia of different vessels. The device may be coated with something which prevents clots (e.g. heparin).

Materials from which the device of the present invention may be constructed include: metals such as nitinol, Elgiloy, stainless steel, and titanium, and bio-compatible plastics, such as PTFE, ePTFE, polyester, silicone, and nylon.

The deflector of the present invention may take alternative shapes such as: round, oval, square, rectangular, elliptical, and edge-scalloped or irregular.

The depth profiles useful in the present invention are: flat, rounded, peaked, onion shaped, tent shaped, parachute shaped, conic, cylindrical, plateau, disc shaped (flat, concave, convex, or concave-convex, spherical or any shape with a supporting protrusion that may extend to the opposite wall of the aortic lumen.)

The device may be single layer, bi-layer, or multi-layer and may be comprised of overlapping or connecting components.

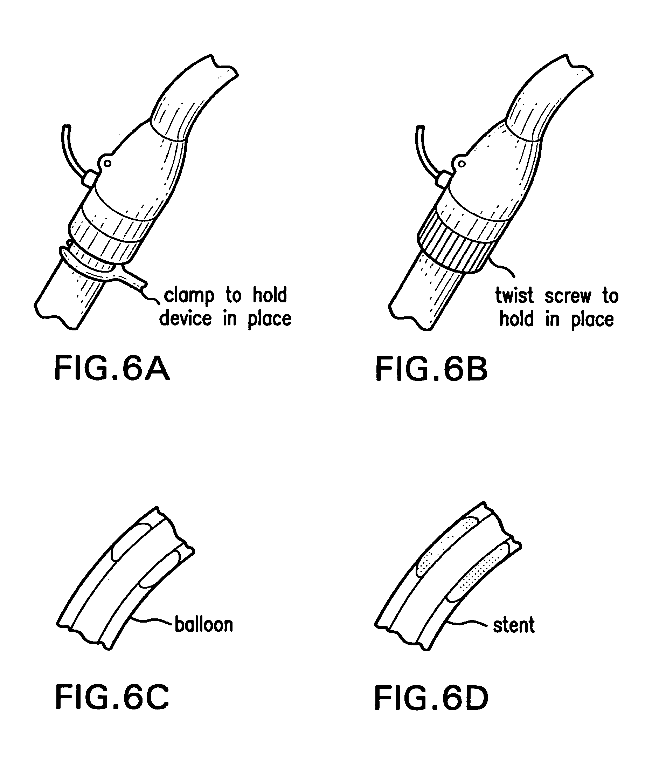

During deployment, the device may be locked in position using a locking mechanism such as is depicted in Figs. 6A-D comprising: a clamp (6A); a twist screw with our without a ratchet (6B); a balloon (6C); or a stent-like sleeve (6D).

Possible methods of deployment of the device include opening an umbrella (with or without struts), overlapping of opening petals (blooming), opening of overlapping elements as in an iris, memory-restoration of a preformed shape, mushrooming, expansion of pores or cells, and release of supporting elements that form the peripheral shape with porous material stretched between.

While the invention has been described in its preferred embodiments, it is to be understood that the words which have been used are words of description rather than of limitation and that changes may be made within the purview of the appended claims without departing from the true scope of the invention in its broader aspects. Rather, various modifications may be made in the details within the scope and range of equivalents of the claims. The inventor further requires that the scope accorded the claims be in accordance with the broadest possible construction available under the law as it exists on the date of filing hereof (and of the application from which this application obtains priority, if any) and that no narrowing of the scope of the appended claims be allowed due to subsequent changes in the law, as such a narrowing would constitute an *ex post facto* adjudication, and a taking without due process or just compensation.

## Claims

1. An embolus deflector for protecting against cerebral embolization comprising a blood flow permeable covering configured to extend over either (a) the ostia of the right common carotid and left common carotid arteries, or (b) the ostia of the brachiocephalic and the left common carotid arteries; **characterized in that** the deflector comprises an expandable support frame, wherein the support frame comprises multiple petals, wherein each petal is formed from wire, wherein porous material extends between the wire of the petals to form the blood flow permeable covering; wherein the support frame has a closed configuration wherein the petals are restrained and an open configuration wherein the petals are opened, wherein in the closed configuration the support frame is sized and configured to be introduced into the aorta, and the support frame is sized and configured to be converted from the closed configuration to the open configuration within the aorta, wherein in the open configuration the support frame is sized and configured to support the blood flow permeable covering within the aorta in a position within the aortic arch and against the aortic wall such that the blood flow permeable covering extends over and covers the left common carotid artery ostium while simultaneously extending over and covering at least one of the ostia of the brachiocephalic artery or of the right common carotid artery, wherein the blood flow permeable covering simultaneously presents a low profile against the aortic wall and within the lumen of the aortic arch to thereby cause minimal resistance to blood flow in the aortic arch by permitting blood to flow through the aortic arch past the blood flow permeable covering without passing therethrough and also to allow sheaths, catheters, or wires to pass through the aortic arch and past the blood flow permeable covering without passing therethrough..

2. The deflector of Claim 1 wherein the deflector is configured for insertion and placement through a catheter in an artery of the arm of a patient.

3. The deflector of Claim 2 wherein the artery is the brachial artery of a patient.

4. The deflector of Claim 1 wherein the deflector is configured for one of:
insertion and placement through a catheter in the femoral artery of a patient; or
insertion and placement directly into the aorta of a patient during an open surgical procedure.

5. The deflector of Claim 1 wherein the deflector is one of:
operative to be inverted prior to withdrawal to trap debris; or operative to be closed prior to withdrawal to trap debris.

6. The deflector of Claim 1 or 5 wherein the periphery is one of:
adapted to seal against the walls of the aortic arch; or supported by a flexible member.

7. The deflector of Claim 6 wherein the periphery is supported by a member constructed from one or more materials selected from the group comprising: nitinol, Elgiloy, stainless steel, plastic, PTFE, ePTFE, polyester, nylon, silicone, and titanium.

8. The deflector of Claim 6 wherein the shape of the periphery is selected from the group comprising: round, oval, square, rectangular, elliptical, edge-scalloped and irregular.

9. The deflector of Claim 8 wherein the profile of the deflector in side view is selected from the group comprising: flat, rounded, peaked, onion shaped, tent shaped, parachute shaped, conic, cylindrical, plateau, disc shaped (flat, concave, convex, or concave-convex, and spherical).

10. The deflector of Claim 8 wherein a supporting member protrudes from the top surface and is arranged to extend to the opposite wall of the aortic lumen when the deflector is in place.

11. The deflector of Claim 1 further comprising a locking means, and which can optionally comprise a locking means selected from the group comprising: a clamp, a twist screw without ratchet, a twist screw with ratchet, a balloon, and a stent-like sleeve.

12. The deflector of claim 11, wherein the frame comprises a supporting protrusion, and wherein the supporting protrusion is sized and configured to simultaneously extend from the blood flow permeable covering to an opposite wall of the aortic lumen when the blood flow permeable covering is positioned within the aorta in a position within the aortic arch and against the aortic wall such that the blood flow permeable covering extends over and covers the left common carotid artery ostium while simultaneously extending over and covering at least one of the ostia of the brachiocephalic artery or of the right common carotid artery, while simultaneously presenting a low profile against the aortic wall and within the lumen of the aortic arch to thereby cause minimal resistance to blood flow in the aorta.

13. The deflector of claim 1, wherein the deflector is deployed from a delivery catheter, the delivery catheter is sized and configured to be advanced into an aortic arch of a patient via a blood vessel of an arm of a patient, and wherein when the support frame is in the open configuration the deflector forms a curved shape, wherein the curved shape is sized and configured to simultaneously cover the ostium of the left common artery and the ostium of the brachiocephalic artery when placed against the aortic wall, while simultaneously presenting a low profile against the aortic wall and within the lumen of the aortic arch to thereby cause minimal resistance to blood flow in the aorta.

14. The deflector of claim 13, wherein the delivery catheter has an internal diameter of about 1.5mm to about 3mm.

15. The deflector of claim 14, wherein the petals expand to assume their open shape to form the curved shape of the deflector when the deflector exits the delivery catheter.

## Patentansprüche

1. Embolus-Deflektor für den Schutz gegen zerebrale Embolisation, der eine für den Blutfluss permeable Abdeckung umfasst, die konfiguriert ist, um sich über entweder (a) die Ostia der rechten Arteria carotis communis und linken Arteria carotis communis oder (b) die Ostia der Arteria brachiocephalica und der linken Arteria carotis communis zu erstrecken; **dadurch gekennzeichnet, dass** der Deflektor einen expandierbaren Stützrahmen umfasst, worin der Stützrahmen mehrere Blätter umfasst, worin jedes Blatt aus Draht gebildet ist, worin sich poröses Material zwischen dem Draht der Blätter erstreckt, um die für den Blutfluss permeable Abdeckung zu bilden; worin der Stützrahmen eine geschlossene Konfiguration aufweist, worin die Blätter zurückgehalten werden, und eine offene Konfiguration aufweist, worin die Blätter geöffnet sind, worin der Stützrahmen in der geschlossenen Konfiguration dimensioniert und konfiguriert ist, um in die Aorta eingeführt zu werden, und der Stützrahmen dimensioniert und konfiguriert ist, um innerhalb der Aorta von der geschlossenen Konfiguration in die offene Konfiguration umgewandelt zu werden, worin der Stützrahmen in der offenen Konfiguration dimensioniert und konfiguriert ist, um die für den Blutfluss permeable Abdeckung innerhalb der Aorta in einer Position innerhalb des Aortenbogens und gegen die Aortenwand zu unterstützen, sodass sich die für den Blutfluss permeable Abdeckung über das Ostium der linken Arteria carotis communis erstreckt und dieses abdeckt, während sie sich gleichzeitig über mindestens eines der Ostia der Arteria brachiocephalica oder der rechten Arteria carotis communis erstreckt und dieses abdeckt, worin die für den Blutfluss permeable Abdeckung gleichzeitig ein niedriges Profil gegen die Aortenwand und innerhalb des Lumens des Aortenbogens darstellt, um dadurch einen minimalen Widerstand gegen den Blutfluss im Aortenbogen zu bewirken, indem das Fließen des Bluts durch den Aortenbogen vorbei an der für den Blutfluss permeablen Abdeckung ermöglicht wird, ohne durch diese hindurch zu gelangen, und um zu erlauben, dass Hülsen, Katheter oder Drähte durch den Aortenbogen und vorbei an der für den Blutfluss permeablen Abdeckung gelangen, ohne durch diese hindurch zu gelangen.

2. Deflektor nach Anspruch 1, worin der Deflektor für die Einführung und Platzierung durch einen Katheter in einer Arterie des Arms eines Patienten konfiguriert ist.

3. Deflektor nach Anspruch 2, worin die Arterie die Arteria brachialis eines Patienten ist.

4. Deflektor nach Anspruch 1, worin der Deflektor für eines der Folgenden konfiguriert ist:
Einführung und Platzierung durch einen Katheter in der Arteria femoralis eines Patienten;
oder
Einführung und Platzierung direkt in die Aorta eines Patienten während eines offenen operativen Eingriffs.

5. Deflektor nach Anspruch 1, worin der Deflektor eines von Folgenden ist:
funktionsfähig, um vor dem Zurückziehen umgewendet zu werden, um Debris einzufangen; oder funktionsfähig, um vor dem Zurückziehen geschlossen zu werden, um Debris einzufangen.

6. Deflektor nach Anspruch 1 oder 5, worin die Peripherie eines von Folgenden ist:
angepasst, um gegen die Wände des Aortenbogens abzudichten; oder gestützt durch ein flexibles Element.

7. Deflektor nach Anspruch 6, worin die Peripherie durch ein Element gestützt wird, das aus einem oder mehreren Materialien konstruiert ist, die aus der Gruppe ausgewählt sind, die Folgende umfasst: Nitinol, Elgiloy, Edelstahl, Kunststoff, PTFE, ePTFE, Polyester, Nylon, Silikon und Titan.

8. Deflektor nach Anspruch 6, worin die Form der Peripherie aus der Gruppe ausgewählt ist, die Folgende umfasst: rund, oval, quadratisch, rechteckig, elliptisch, am Rand rundgezackt und unregelmäßig.

9. Deflektor nach Anspruch 8, worin das Profil des Deflektors in Seitenansicht aus der Gruppe ausgewählt ist, die Folgende umfasst: eben, gerundet, spitz, zwiebelförmig, zeltförmig, fallschirmförmig, konisch, zylindrisch, plateauförmig, scheibenförmig (eben, konkav, konvex oder konkav-konvex und kugelförmig).

10. Deflektor nach Anspruch 8, worin ein stützendes Element aus der oberen Oberfläche herausragt und angeordnet ist, um sich zur gegenüberliegenden Wand des Aortenlumens zu erstrecken, wenn sich der Deflektor an der Stelle befindet.

11. Deflektor nach Anspruch 1, der weiter ein Arretiermittel umfasst und der optional ein Arretiermittel umfasst, das aus der Gruppe ausgewählt ist, die Folgende umfasst:
eine Klemme, eine Schraubverbindung ohne Ratsche, eine Schraubverbindung mit Ratsche, einen Ballon und eine Stent-ähnliche Hülse.

12. Deflektor nach Anspruch 11, worin der Rahmen eine stützende Vorwölbung umfasst und worin die stützende Vorwölbung dimensioniert und konfiguriert ist, um sich gleichzeitig von der für den Blutfluss permeablen Abdeckung zu einer gegenüberliegenden Wand des Aortenlumens zu erstrecken, wenn die für den Blutfluss permeable Abdeckung innerhalb der Aorta in einer Position innerhalb des Aortenbogens und gegen die Aortenwand positioniert ist, sodass sich die für den Blutfluss permeable Abdeckung über das Ostium der linken Arteria carotis communis erstreckt und dieses abdeckt, während sie sich gleichzeitig über mindestens eines der Ostia der Arteria brachiocephalica oder der rechten Arteria carotis communis erstreckt und dieses abdeckt, während sie gleichzeitig ein niedriges Profil gegen die Aortenwand und innerhalb des Lumens des Aortenbogens darstellt, um dadurch einen minimalen Widerstand gegen den Blutfluss in der Aorta zu bewirken.

13. Deflektor nach Anspruch 1, worin der Deflektor aus einem Einführkatheter eingesetzt wird, wobei der Einführkatheter dimensioniert und konfiguriert ist, um in einen Aortenbogen eines Patienten über ein Blutgefäß eines Arms eines Patienten vorgeschoben zu werden, und worin, wenn der Stützrahmen in der offenen Konfiguration vorliegt, der Deflektor eine gebogene Form bildet, worin die gebogene Form dimensioniert und konfiguriert ist, um gleichzeitig das Ostium der linken Arteria carotis communis und das Ostium der Arteria brachiocephalica abzudecken, wenn sie gegen die Aortenwand platziert wird, während sie gleichzeitig ein niedriges Profil gegen die Aortenwand und innerhalb des Lumens des Aortenbogens darstellt, um dadurch einen minimalen Widerstand gegen den Blutfluss in der Aorta zu bewirken.

14. Deflektor nach Anspruch 13, worin der Einführkatheter einen Innendurchmesser von etwa 1,5 mm bis etwa 3 mm aufweist.

15. Deflektor nach Anspruch 14, worin sich die Blätter erstrecken, um ihre offene Form anzunehmen, um die gebogene Form des Deflektors zu bilden, wenn der Deflektor aus dem Einführkatheter austritt.

## Revendications

1. Déflecteur d'emboles offrant une protection contre l'embolisation cérébrale, qui comprend une chape perméable au flux sanguin configurée de manière à s'étendre soit (a) sur les ostia des artères carotides communes droite et gauche, soit (b) sur les ostia du tronc artériel brachio-céphalique et de l'artère carotide commune gauche ; **caractérisé en ce que** le déflecteur comprend une armature de support expansible, ladite armature de support comprenant de multiples pétales chacune formée à partir d'un fil métallique, un matériau poreux s'étendant entre le fil métallique des pétales pour former la chape perméable au flux sanguin ; dans lequel l'armature de support a une configuration fermée dans laquelle les pétales sont retenues et une configuration ouverte dans laquelle les pétales sont déployées, l'armature de support en configuration fermée ayant des dimensions et une conformation qui permettent son introduction dans l'aorte et l'armature de support ayant des dimensions et une conformation qui permettent de passer de la configuration fermée à la configuration ouverte à l'intérieur de l'aorte, l'armature de support en configuration ouverte ayant des dimensions et une conformation qui permettent de maintenir la chape perméable au flux sanguin positionnée dans l'aorte à l'intérieur de la crosse de l'aorte et contre la paroi aortique, de sorte que la chape perméable au flux sanguin s'étend sur l'ostium de l'artère carotide commune gauche et le recouvre et simultanément s'étend sur l'ostium du tronc artériel brachio-céphalique et/ou de l'artère carotide commune droite et le(s) recouvre, dans lequel la chape perméable au flux sanguin présente simultanément un profil bas contre la paroi aortique et dans la lumière de la crosse de l'aorte, produisant ainsi une résistance minimale au flux sanguin dans la crosse aortique en permettant au sang de s'écouler dans la crosse de l'aorte jusqu'en aval de la chape perméable au flux sanguin sans passer au travers de celle-ci et permettant aussi à des gaines, cathéters ou sondes d'être poussés au travers de la crosse de l'aorte jusqu'en aval de la chape perméable au flux sanguin sans passer au travers de celle-ci.

2. Déflecteur selon la revendication 1, dans lequel le déflecteur est configuré pour être inséré et posé au travers d'un cathéter dans une artère du bras d'un patient.

3. Déflecteur selon la revendication 2, dans lequel l'artère est l'artère brachiale d'un patient.

4. Déflecteur selon la revendication 1, dans lequel le déflecteur est configuré pour se prêter à l'une des procédures suivantes :
insertion et pose au travers d'un cathéter dans l'artère fémorale d'un patient ; ou
insertion et pose directement dans l'aorte d'un patient lors d'une intervention chirurgicale à ciel ouvert.

5. Déflecteur selon la revendication 1, dans lequel le déflecteur soit :
fonctionne de manière à être inversé avant le retrait pour piéger les débris ; soit fonctionne de manière à être fermé avant le retrait pour piéger les débris.

6. Déflecteur selon la revendication 1 ou 5, dans lequel la périphérie est l'une des suivantes :
adapté pour assurer l'étanchéité le long des parois de la crosse de l'aorte ; ou
soutenu par un élément flexible.

7. Déflecteur selon la revendication 6, dans lequel la périphérie est soutenue par un élément constitué d'un ou de plusieurs matériaux sélectionnés dans le groupe comprenant les suivants : Nitinol, Elgiloy, acier inoxydable, plastique, PTFE, ePTFE, polyester, nylon, silicone et titane.

8. Déflecteur selon la revendication 6, dans lequel la forme de la périphérie est sélectionnée dans le groupe comprenant les suivantes : ronde, ovale, carrée, rectangulaire, elliptique, à lisière échancrée et irrégulière.

9. Déflecteur selon la revendication 8, dans lequel le profil du déflecteur en incidence latérale est sélectionné dans le groupe comprenant les suivants : plat, arrondi, pointu, en forme d'oignon, en forme de tente, en forme de parachute, conique, cylindrique, en plateau, en forme de disque (plat, concave, convexe ou concave-convexe et sphérique).

10. Déflecteur selon la revendication 8, dans lequel un élément de support fait saillie à la surface supérieure et est disposé de manière à s'étendre jusqu'à la paroi opposée de la lumière aortique quand le déflecteur est en place.

11. Déflecteur selon la revendication 1, qui comprend en outre un moyen de blocage et qui peut en option comprendre un moyen de blocage sélectionné dans le groupe consistant en les suivants : bride de fixation, vis torsadée sans cliquet, vis torsadée avec cliquet, ballonnet et manchon de type stent.

12. Déflecteur selon la revendication 11, dans lequel l'armature comprend une saillie porteuse et dans lequel la saillie porteuse présente des dimensions et une conformation lui permettant de simultanément s'étendre de la chape perméable au flux sanguin jusqu'à la paroi opposée de la lumière aortique quand la chape perméable au flux sanguin est positionnée dans l'aorte à l'intérieur de la crosse de l'aorte et contre la paroi aortique, de sorte que la chape perméable au flux sanguin s'étend sur l'ostium de l'artère carotide commune gauche et le recouvre et simultanément s'étend sur l'ostium du tronc artériel brachio-céphalique et/ou de l'artère carotide commune droite et le(s) recouvre, tout en présentant simultanément un profil bas contre la paroi aortique et dans la lumière de la crosse de l'aorte, produisant ainsi une résistance minimale au flux sanguin dans l'aorte.

13. Déflecteur selon la revendication 1, dans lequel le déflecteur est déployé à partir d'un cathéter de pose, dans lequel le cathéter de pose présente des dimensions et une conformation qui permettent de le faire avancer dans la crosse de l'aorte d'un patient par le biais d'un vaisseau sanguin du bras du patient et dans lequel le déflecteur adopte une forme incurvée quand l'armature de support est en configuration ouverte, la forme incurvée ayant des dimensions et une conformation lui permettant de simultanément couvrir l'ostium de l'artère carotide commune gauche et l'ostium du tronc artériel brachio-céphalique quand elle est positionnée contre la paroi aortique tout en présentant simultanément un profil bas contre la paroi aortique et dans la lumière de la crosse de l'aorte, produisant ainsi une résistance minimale au flux sanguin dans l'aorte.

14. Déflecteur selon la revendication 13, dans lequel le diamètre interne du cathéter de pose va d'environ 1,5 mm à environ 3 mm.

15. Déflecteur selon la revendication 14, dans lequel les pétales se déplient pour adopter leur configuration ouverte pour créer la forme incurvée du déflecteur quand le déflecteur est libéré du cathéter de pose.
